# EUROPEAN PATENT SPECIFICATION

(11) **EP 0 159 727 B1**
(45) Date of publication and mention of the grant of the patent: **10.03.1993**
(21) Application number: 85105214.2
(22) Date of filing: 29.04.1985
(51) Int. Cl.: G01N 33/52, G01N 33/72

(54) **Analytical element for analysis of whole blood sample**
Analytisches Element zur Analyse einer Vollblutprobe
Elément de test pour analyse d'un échantillon de sang entier

(30) Priority: 27.04.1984 JP 87460/84; 27.04.1984 JP 87461/84; 17.10.1984 JP 217761/84
(43) Date of publication of application: 30.10.1985
(73) Proprietor: FUJI PHOTO FILM CO., LTD., Kanagawa 250-01 (JP)
(72) Inventor: Masuda, Nobuhito, Asaka-shi Saitama (JP); Igarashi, Takeshi, Asaka-shi Saitama (JP); Katsuyama, Harumi, Asaka-shi Saitama (JP)
(74) Representative: Werner, Hans-Karsten, Dr.Dipl.-Chem.

(56) References cited:
- EP-A- 0 115 873
- GB-A- 2 052 057
- US-A- 3 992 158
- US-A- 4 042 335
- US-A- 4 069 017
- US-A- 4 256 693

## Description

### BACKGROUND OF THE INVENTION

### Field of The Invention

This invention relates to an analytical element for the analysis of a whole blood sample. More particularly this invention relates to an analytical element capable of analyzing an analyte without being influenced by the fluctuation of hematocrit values in whole blood in the analysis of the whole blood sample.

### Description of Prior Arts

Heretofore, in the biochemical test of blood, there has been used a method wherein plasma or serum (both are hereinafter referred to simply as "plasma" unless otherwise stated) is separated from whole blood and employed as test sample, since the whole blood collected from patients can not be directly used as test sample. Further, the analysis of an analyte in plasma has been generally performed by wet colorimetric chemical analysis using test tubes and liquid reagents
Since both the separation of plasma and the wet chemical analysis are indispensable stages in the conventional biochemical test, the procedure for preparation and operation are complicated, and much skill and considerable time are required. In the conventional biochemical test, therefore, it is difficult to meet doctors' requirements to utilize immediately use the test results for diagnosis during an interview with patients.

Until now, attempts of simplifying or directly conducting the biochemical test have been made. Most of them are such methods that whole blood is used as a test sample without carrying out the plasma separation as a preliminary stage and a sheet-form dry analytical element (for example an integral multilayer analytical element) having a reagent or a blood cell-filtering function previously incorporated therein is used in place of conducting the wet chemical analysis.

An example of such a dry method using whole blood as a sample includes a method and an analytical element disclosed in Japanese Patent Provisional Publication No. 49(1974)-11395. In this method a disc type element comprising four layers composed of two sheets of porous glass filters, a thin-filmy filter and a detecting reagent-containing cellulose acetate sheet in a superposed form in this order from the top is used as the analytical element. When the whole blood is dropped on this element, leucocyte, erythrocyte and other material components are removed, and plasma is admixed with the reagent to effect a color forming reaction on the cellulose acetate sheet.

Other example thereof includes a method and an element for separating plasma from whole blood disclosed in Japanese Patent Provisional Publication No. 57(1982)-53661 wherein a specific glass filter layer is used as a blood cell filter. For the analysis of whole blood, this filter layer may be put on a usual multilayer diagnostic material, for example, a testpaper type diagnostic material impregnated with a reagent (disclosed in, for example, Japanese Patent Provisional Publication No. 54(1979) -151096) to form an analytical element.

These methods and elements are effective in separating material components such as blood cell, but there is a disadvantage that the amount of plasma to react with an elemental reagent fluctuates for every samples, since the volume of blood cell portion in whole blood in relation to the hematocrit value (hereinafter referred to as Hct value) inherently flucturates. The whole blood consists of the blood cell part and the plasma part. The Hct value is not constant for different samples, and varies between about 20% and about 70% from person to person. For example, when each of whole blood having a Hct value of 30% and that having a Hct value of 50% is used as a sample and 100 µℓ of each sample is dropped onto the analytical element, the amount of plasma in the former sample to react with the reagent is 70 µℓ and the amount of plasma in the latter sample is 50 µℓ. Thus, the results are deviated from the principle of quantitative analysis. Further, the amount of analyte in the plasma part usually much differs from that in the blood cell part and this fact also disturbs the desired quantitative analysis.

Accordingly, the test results are not reliable when whole blood is directly used as a sample in the analysis of whole blood with the known analytical elements.

There is disclosed in Japanese Patent Publication No. 53(1978)-21667 and Japanese Patent Provisional Publication No. 55(1980)-90859 an integral multilayer analytical element having a porous spreading layer (hereinafter referred to as spreading layer) capable of spreading a whole blood sample, i.e., metering the spotted liquid sample. The term "spreading layer capable of metering a liquid sample" herein used refers to a layer having a function capable of spreading a liquid droplet in such a manner that the spread area of the liquid is approximately in proportion to the amount of the liquid when the liquid droplet is spotted thereon and further having a function capable of supplying the liquid to a layer provided under the spreading layer. For example, when the amount of the spotted liquid is 5 µℓ and the area of the liquid to be spread is 0.5 cm², the 10 µℓ of the spotted liquid should be spread over 1.0 cm² in area, and similarly 15 µℓ of the spotted liquid should be spread over 1.5 cm² in area. This function is called metering effect or spreading. The multilayer analytical elements having such a spreading layer have an advantage in that even when different amounts of liquid samples are spotted on the surface of the spreading layer, the liquid amount to be introduced into the spreading layer per se per unit area thereof and the liquid amount to be supplied to lower layers such as a reagent layer are always kept constant by the above-mentioned metering effect.

The spreading layer disclosed in the aforementioned patent specifications has a liquid sample-metering effect on the whole amount of the whole blood sample, so the Hct value is not taken into consideration. The material components such as blood cell hardly penetrates into the reagent layer so that quantitative analysis is not considered satisfactory with respect to the resulting values.

In order to perform quantitative analysis directly using whole blood as a sample in a dry multilayer analytical element, the dry analytical method should give the same analytical value as that obtained by a wet analytical method using plasma as a sample. Thus, th method should be capable of analyzing the amount of an analyte in the plasma part of whole blood with high accuracy.

There is disclosed in Japanese Patent Provisional Publication No. 55(1980)-164356 (GB-2 052 057) a multilayer analytical element using a combination of a gelatin layer and a fabric to spread whole blood spotted thereon and to perfectly filter the blood cell part thereof. The element is constructed such that the spreading layer gives a metering effect to plasma. However, when an analyte is a high molecular material, there arise problems that the blood cell part in whole blood interferes with the migration and the diffusion of the analyte and the analyte hardly reaches the reagent layer so that analytical accuracy is low.

US Patent 4,256,693 discloses an analytical element comprising a filter layer capable of removing formed components from the blood, a water-proof layer having at least one small opening therein, a porous spreading layer and a reagent layer. Filter layer and spreading layer are separated from each other by the intervening water-proof layer.

US Patent 4,042,335 teaches a multilayer analysis sheet comprising a reagent-containing spreading layer and a combination of a spreading layer and a reagent-containing layer, a radiation-blocking layer and a light transmissive water-impermeable support. On the spreading layer no filter layer is arranged.

US Patent 4,069,017 describes a spreading layer - which may contain a reagent - a reagent layer and a radiation transmissive support. Furthermore a radiation-blocking layer is used. No filter layer arranged on the spreading layer is described.

### SUMMARY OF THE INVENTION

It is an object of the present invention to provide a dry analytical element capable of analyzing an analyte with high accuracy in the analysis using whole blood irrespective of fluctuation of hematocrit value.

It is another object of the present invention to provide a dry analytical element capable of analyzing a high-molecular analyte with high accuracy in the analysis using whole blood irrespective of fluctuation of hematocrit value.

The present invention provides an analytical element for the analysis of a whole blood sample; said element comprising a blood cell- filtering layer, a spreading layer capable of metering a liquid sample, a detecting layer containing at least one reagent layer, and a water-impermeable, light-transmissive support; which are positioned in this order.

The present invention also provides an analytical element for the analysis of a whole blood sample comprising a blood cell-filtering layer, a spreading layer capable of metering a liquid sample which contains an analytical reagent and a water-impermeable, light-transmissive support; which are positioned in this order.

The analytical element for the analysis of whole blood according to the present invention is one which allows whole blood to be directly used as a sample and enables an analyte present therein to be analyzed in a dry method. Accordingly, the analytical element of the present invention shows an valuable advantage in that the analyte can be quantitatively analyzed with high accuracy merely by spotting a droplet of a whole blood sample on the element with no requirement of a complicated operation for plasma separation.

In the analytical element of the present invention, the blood cell-filtering layer is placed closely on the surface of the spreading layer capable of metering the liquid sample so that blood cells are filtered off before liquid sample is metered on the surface of the spreading layer. Hence, only plasma is subjected to the liquid sample metering so that the resulting value correctly shows the amount of an analyte in the plasma part, even though whole blood is used as the sample. Further, the blood cell part does not interfere with the migration and the diffusion of the analyte so that whole blood can be directly analyzed as the sample even when the analyte is a high-molecular one or in the combined form with a high-molecular material.

The analytical element of the present invention can be composed of such a structure that a porous material as a blood cell-filtering layer is placed on the spreading layer and the liquid sample is supplied therethrough to the spreading layer, but in such a structure, the analysis of analyte in the plasma of whole blood can be made with high accuracy without detriment to the liquid sample metering function of the spreading layer. Accordingly, simplifying and immediately conducting an analytical operation can be made possible using the analytical element of the present invention as compared with the conventional analytical method using a combination of plasma separation and wet analysis. Further, the analytical element can meet the doctors' requirement to immediately utilize the test results for diagnosis during an interview with patients.

### BRIEF DESCRIPTION OF THE DRAWINGS

Figure 1 is a schematic view showing the layer construction of a conventional multilayer analytical element.

Figure 2 is a schematic view showing a representative layer construction of the analytical element for whole blood analysis according to the present invention.

### DETAILED DESCRIPTION OF THE INVENTION

The spreading layer capable of metering a liquid sample, which can be used in the present invention is known and can be chosen from conventional ones according to the type of analytes and analytical conditions. For example, the non-fibrous isotropic porous medium layer (e.g. membrane filter and brush polymer layer) and the fibrous (anisotropic) porous medium layer (e.g. woven fabric and knitted fabric) disclosed in Japanese Patent Publication No. 53(1978)-21677 can be used.

However, in the case that the spreading layer and the blood cell-filtering layer are used in an integrated form, it is preferred to use a spreading layer composed of a woven cloth or a knitted cloth as described in more detail hereinafter.

Examples of the woven fabrics (woven cloth) which can be used for the spreading layer include those disclosed in Japanese Patent Provisional Publication No. 55(1980)-164356 and No. 57(1982)-66359. Among the woven cloth, plain weave fabrics made of warp and weft are preferred. Among plain waven fabrics, thin cloth, muslin, broadcloth and poplin are preferred.

Examples of yarns for woven cloth include those composed of the same materials as those constituting knitted cloths as described in more detail hereinafter. Any of filament yarn and spun yarn (twist yarn) can be used, and the spun yarn is preferred. The yarn diameter of the woven cloth is generally in the range of about 20S to about 150S, preferably about 40S to about 120S in terms of cotton spinning yarn count or in the range of about 35 to about 300D, preferably about 45 to about 130D in terms of silk thread denier. The thickness of the woven cloth is generally in the range of about 100 to about 500 µm, preferably about 120 to 350 µm. The voids of the woven cloth are generally in the range of about 40 to about 90%, preferably about 50 to about 85%.

Examples of the knitted cloths which can be used for the spreading layer include many kinds of knitted cloths, among which warp knitted fabric and weft knitted fabric are preferred. Examples of the warp knitted fabrics include single atlas knitted cloth, tricot knitted cloth, double tricot knitted cloth, milanese knitted cloth and rashar knitted cloth. Examples of the weft knitted fabrics include plain weave knitted cloth, pearl knitted cloth, rubber knitted cloth, and double face knitted cloth. Examples of the yarns for knitted fabrics include yarns of natural fibers such as cotton, silk and wool; yarns composed of fine fibers or single fibers of regenerated cellulose (e.g. viscose rayon and cupra), semisynthetic organic polymer (e.g. cellulose diacetate and cellulose triacetate), synthetic organic polymer (e.g. polyamide such as nylon, acetalated polyvinyl alcohol such as vinylon, polyacrylonitrile, polyethylene terephthalate, polyethylene, polypropylene and polyurethane), and yarns composed of fiber blends of a natural fiber and a regenerated cellulose or a semisythetic or synthetic organic polymer fiber. Any of filament yarn and spun yarn can be used, and spun yarn is preferred. The diameter of the yarn for knitted fabric is generally in the range of from about 40 to 150S, preferably about 60 to about 120S in terms of cotton spinning yarn count, or in the range of about 35 to about 130D, preferably about 45 to about 90D in terms of silk thread denier. The number of knitting gauge of the knitted fabric is generally in the range of about 20 to about 50. The thickness of the knitted fabric is generally in the range of about 100 to about 600 µm, preferably about 150 to about 400 µm. The voids of the knitted fabric are generally in the range of about 40 to about 90%, preferably about 50 to about 85%. The warp knitted fabric, tricot knitted cloth, rashar knitted cloth, milanese knitted cloth and double tricot knitted cloth are preferred, because shrinkage in the wale's direction is small, the operation in the lamination stage of knitted goods is easy and the stitches are not easlity loosened during cutting.

Other functions in addition to the spreading function of metering the liquid sample can be given to the spreading layer of the analytical element of the present invention. For example, a detecting reagent can be incorporated into the spreading layer to carry out the analytical reaction in the spreading layer. Such modifications are also included within the scope of the present invention.

In a preferred embodiment of the present invention, the spreading layer having the liquid sample-metering function is incorporated into an integral multilayer analytical element. Examples of the structure of such integral multilayer analytical element containing said spreading layer are disclosed in Japanese Patent Publication No. 53(1978)-21667 and Japanese Patent Provisional Publication No. 55(1980)-164356.

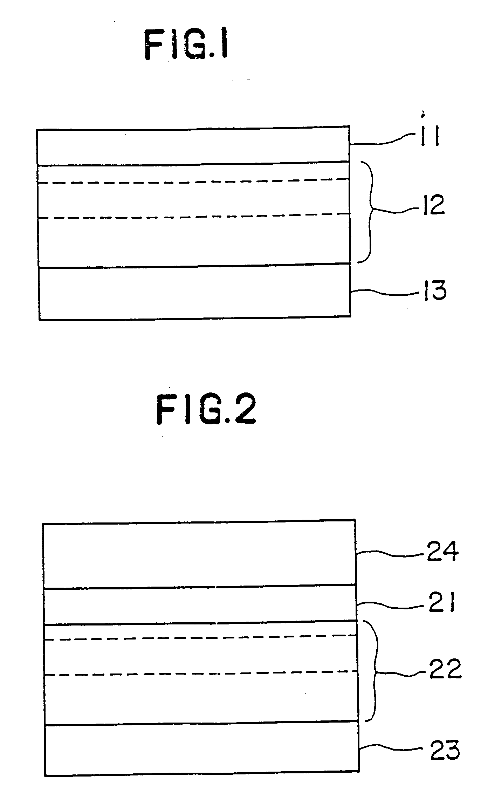

Figure 1 shows an embodiment illustrating the basic structure of the conventional integral analytical element, wherein the uppermost layer (outermost layer) is the spreading layer 11, the lowermost layer is the water-impermeable, light-transmissive (transparent) support 13, and optionally a detection layer 12 is interposed there-between. The detection layer contains at least one reagent layer. However, a reagent may be incorporated in the spreading layer to combine the detection layer with the spreading layer into one layer. In addition to the reagent layer, the detection layer may be composed of any of a reaction layer, a reaction-detection layer, a filter layer, a semipermeable membrane layer, a barrier layer, a light-blocking layer, a light-reflecting layer, a water-absorbing layer, etc. The number of the layers in the detection layer may be one layer or more without specific limitation. One layer in the detection layer may have one function, or one layer may have two or more functions such as a light-blocking material having light reflecting property is incorporated in the reaction layer to enhance the stability of white-reflection density during reflection photometry.

The integral multilayer analytical elements containing the liquid sample-metering function are commercially available in the form of a slide for every analytes in blood. These elements as such may be used in the preparation of the analytical element of the present invention. If desired, these elements may be modified for the incorporation to the analytical element of the invention. Alternatively, an integral multilayer analytical ecements containing a spreading layer having the liquid sample-metering function may be prepared in consideration of the analyte and analytical conditions from the disclosures of literatures.

Figure 2 shows diagrammatically an embodiment of an analytical element for the analysis of whole blood sample according to the present invention, wherein a blood cell-filtering layer is provided on a spreading layer in such a manner that the blood cell-filtering layer 24 is provided on the spreading layer 21 of a multilayer analytical element comprising the spreading layer 21, a detection layer 22 and a water-impermeable, light-transmissive (transparent) support 23. Each of the spreading layer 21, the detection layer 22 and the support 23 corresponds to each of the spreading layer 11, the detection layer 12 and the water-impermeable, light-transmissive (transparent) support 13 shown in Figure 1 which shows the basic structure of the integral multilayer analytical element. Further, the composite structure of the members 21, 22 and 23 shown in Figure 2 also corresponds to the integral multilayer analytical element composed of the members 11, 12 and 13 shown in Figure 1.

Materials for the blood cell-filtering layer of the invention are filter mediums having function capable of removing blood cell from whole blood. Example of such filter mediums include glass fiber filter, membrane filter and cotton-wool fiber products. Although any of them can be used for the blood cell-filtering layer of the invention, glass fiber filter is particularly preferred, because the blood cells can be filtered off rapidly and completely by the use of glass fiber filter.

The glass fiber filters are commercially available, and the desired filter having satisfactory blood cell-filtering performance can be chosen from the articles on the market, taking fiber length, fiber diameter, voids and absorptive power into consideration. The glass fiber filter may be prepared by known technique. Examples of such glass fiber filters include those disclosed in Japanese Patent Provisional Publication No. 57(1982)-53661, and GA-100 (about 450µm in thickness), GA-200 (about 750 µm in thickness) and GB-100R (about 400 µm in thickness) marketed by Toyo Filter Paper Co., Ltd., Japan.

It is necessary that after whole blood is filtered, the color of erythrocyte remained in the blood cell-filtering layer is made invisible in reflection photometry from the side of the transparent support. For this purpose, the filtering layer may be removed during photometry. However, it is preferred that a light-blocking material such as a dye or a pigment is incorporated into the whole or the bottom of the blood cell-filtering layer to impart a property as a light-blocking layer to a part or the whole of the filtering layer. For example, when the bottom of the filtering layer is filled with a white pigment such as titanium dioxide or barium sulfate, the filter covers the color of erythrocyte and plays a role as a reflecting layer in reflection photometry.

A hemolysis inhibitor or an anticoagulant may be incorporated in the blood cell-filtering layer to prevent plasma from being stained by blood cells. If desired, other reagent such as coagulation promoter may be added.

The blood cell-filtering layer has such a thickness that blood cell-filtering performance can be satisfactorily exhibited and the analytical operation can be terminated within the desired time. The thickness of the filtering layer is preferably from 100 to 2,000 µm, particularly 150 to 1,000 µm. The size of the blood cell-filtering layer is such that the whole surface of the spreading layer can be covered therewith, or only a part of the spreading layer may be covered by a piece of the blood cell-filtering layer (namely, a patch). However, when the size of the blood cell-filtering layer is too small, blood cells may leak out of the side of the filtering layer. In such a case, it is necessary to seal the side of the patch with a hydrophobic material. When the blood cell-filtering layer has such a size that the whole surface of the spreading layer is covered therewith, the surface of the blood cell-filtering layer may be covered with a hydrophobic ink by means of screen printing while the part to be spotted is left uncovered.

It is necessary that the blood cell-filtering layer is placed uniformly, closely on the spreading layer such that the liquid sample can smoothly pass through the boundary surface between both layers without causing an interruption of the liquid flow. The provision of the filtering layer closely on the spreading layer can be effected by applying electrostatic adhesion, or by pressing the outer edge of the filtering layer against the surface of the spreading layer and bonding them to each other in such a manner that the bounded area does not interfere with the flow of the liquid sample. Alternatively, the spreading layer and the blood cell-filtering layer may be combined together to form an integral structure depending on the material of the spreading layer.

One of several methods for combining the spreading layer with the filtering layer together into an integral type is that the blood cell-filtering layer is laminated on the preformed spreading layer by a paper-making technique. A simple method is that a material of the spreading layer is first chosen to form a relatively dense spreading layer. Then a material of the bulky blood cell-filtering layer having a density lower than that of the spreading layer is chosen and the blood cell-filtering layer is laminated on the spreading layer by a paper-making technique to form an integral structure. For example, in the case that a broadcloth is chosen as a material of the spreading layer, a slurry is put on said broadcloth by a paper-making technique, whereby the blood cell-filtering layer can be easily obtained, said slurry containing a fiber dispersed therein, said fiber being equal to, or larger or longer than the fiber constituting a yarn from which said broadcloth is formed.

The analysis of an analyte in whole blood using the integral multilayer analytical element having the blood cell-filtering layer according to the present invention can be generally carried out in such a manner that a given amount of whole blood is spotted on the surface of the blood cell-filtering layer and incubated for a given period of time, a color density is measured from the side of the transparent support by means of reflection photometry and the amount of the analyte present in the plasma of the whole blood is calculated using a calibration curve previously prepared.

Preferably, whole blood is spotted on the surface of the blood cell-filtering layer in such an amount that several µℓ to several tens of µℓ of plasma is filtered through said layer and reaches the lower spreading layer, taking the range of fluctuation in Hct value into consideration.

For example, assuming that the Hct values of the sample vary within 30 and 60%, the amount of plasma in 30 µℓ of whole blood theoretically ranges from 21 to 12 µℓ. The amount of plasma reaching the spreading layer is the remainder after an amount remaining in the filtering layer is deducted from the total amount.

It is preferred that when a glass fiber filter is used, the whole blood sample is spoted so as to supply a sufficient amount of plasma to the spreading layer in such an amount that plasma in an excess amount of at least 50% more than that of plasma to be absorbed by the blood cell-filtering layer, reaches the spreading layer, or the blood cell-filtering layer is constructed of a structure such that plasma in the above-mentioned amount can be filtered through the layer.

The analytical element of the present invention can be used for any analytes without specific limitation, and an optionally selected analyte in whole blood can be subjected to quantitative analysis using the analytical element of the present invention. Particularly, the analytical element of the present invention is suitable for use in the analysis of high-molecular analytes such as saccharide, protein and enzyme, and particularly a difficultly diffusible analyte such as bilirubin. The type of reagents and the structure of the multilayer analytical element to match with the analyte are already known, and the known technique can be applied to the preparation of the analytical element for purpose analysis as stated above.

The following examples further illustrate the present invention.

### Example 1 (Reference Example)

According to the examples described in Japanese Patent Publication No. 57(1982)-28227 and Japanese Patent Provisional Publication No. 59(1984)-54962, a reagent layer (thickness: 20µm) and a reflecting layer containing TiO₂ (thickness: 7 µm) were laminated on a transparent polyethylene terephthalate support (thickness: 180µm). Further, a membrane filter FM-300 (thickness: 150µm, minimum pore diameter: 3 µm, voids: average 85%, manufactured by Fuji Photo Film Co., Ltd.) having liquid sample-metering function as a spreading layer was laminated thereon under pressure to prepare an integral multilayer analytical element for quantitative determination of glucose content. Each of the coating solutions for the above regent layer and reflecting layer had the following composition.

| Reagent layer-coating solution | |
|---|---|
| Deionized gelatin | 2,000 g. |
| Glucose oxidase | 15,000 IU |
| Peroxidase | 25,000 IU |
| 4-Aminoantipyrine | 12 g. |
| 1,7-Dihydroxynaphthalene | 5 g. |
| Polyoxyethylene nonylphenyl ether | 2 g. |

| Reflecting layer-coating solution | |
|---|---|
| Deionized gelatin | 10 g. |
| Fine TiO₂ powder | 80 g. |
| Polyoxyethylene nonylphenyl ether | 2 g. |

A glass fiber GA-200(thickness: about 750 µm, voids: about 90%, manufactured by Toyo Filter Paper Co., Ltd.,) was placed closely on the overall surface of the spreading layer of the above multilayer analytical element to obtain the analytical element for the analysis of whole blood according to the present invention. The following experiments were then carried out.

As the sample there was used whole blood which had an Hct value of 35% and in which plasma contained glucose at a concentration of 122 mg/dℓ. Under variation of the amount of whole blood to be spotted on the analytical element, the spread area on the reagent layer was measured, and color density was also measured through reflection photometry (measurement wavelength: 510 nm). The results are shown in Table 1.

**Table 1**

| Amount of whole blood (µℓ) | Area of colored surface, (cm³) | Color density (OD) |
|---|---|---|
| 25 | 0.90 | 0.812 |
| 30 | 1.05 | 0.808 |
| 35 | 1.20 | 0.810 |

It is evident from the above results that the liquid sample-metering function of the spreading layer is not reduced at all even when the blood cell-filtering layer is placed on the spreading layer and the sample solution is supplied therethrough to the spreading layer.

### Example 2

An integral multilayer analytical element for the quantitative determination of the whole bilirubin.

A water-absorbing layer-1 (dry film thickness: 15 µm) and a water-absorbing layer-2 (dry film thickness: 10 µm) were formed on a transparent polyethylene terephthalate support (thickness: 180 µm) in this sequence. A cotton cloth (blood woven cloth woven made of No. 100 count two folded yarn) treated with a surfactant was wet-laminated on the water-absorbing layer to form a matrix of a porous regent layer. Further, said matrix of the porous reagent layer was coated and thereby impregnated with a reagent layer-coating solution I and a reagent layer-coating solution II in turn to prepare an integral multilayer analytical element for use in the assay of the whole bilirubin. Each of the coating solutions for the water-absorbing layers I and II and the reagent layer-coating solutions I and II had the following composition.

| Coating solution for water-absorbing layer-I | |
|---|---|
| Polyvinyl alcohol (a degree of saponification: 88%, viscosity of 4% aqueous solution at 20^{o}C: 5 cps) | 100 g. |
| 50% Aqueous solution of poly (2-hydroxy-3-oxypropylene) -n-nonylphenyl ether | 5 g. |
| Water | 895 mℓ. |

| Coating solution for water-absorbing layer-II | |
|---|---|
| Polyvinyl alcohol (a degree of saponification: 99%, viscosity of 4% aqueous solution at 20^{o}C: 30 cps) | 80 g. |
| 50% Aqueous solution of poly (2-hydroxy-3-oxypropylene) -n-nonylphenyl ether | 5 g. |
| Water | 915 mℓ. |

| Reagent layer-coating solution I | |
|---|---|
| 7-(2,3-Dihydroxypropyl) theophylline [479-18-5] | 150 g. |
| Sulfosalicylic acid | 30 g. |
| Hydroxypropyl methacrylate-N-(α-sulfomethyl-α-methylethyl) acrylamide (6:4) copolymer (1,5% aqueous solution) | 1,000 g. |
| 2,4-Dichlorobenzene diazonium sulfosalicylate | 1.9 g. |

| Reagent layer-coating solution II | |
|---|---|
| Fine titanium oxide powder | 55 g. |
| 7-(2,3-Dihydroxypropyl) theophylline [479-18-5] | 150 g. |
| 50% Aqueous solution of poly (2-hydroxy-3-oxypropyl)-n-nonylphenyl ether | 10 g. |
| Water | 1,000 mℓ. |

A glass fiber filter GB-100R (a disc of 6 mm in diameter, thickness: about 400 µm, voids: about 90%, manufactured by Toyo Filter Paper Co., Ltd.,) was placed in a frame to form a blood cell-filtering layer closely on the spreading layer of the above integral multilayer analytical element for the assay of the whole bilirubin. Thus, an analytical element for the analysis of whole blood according to the present invention was prepared.

30 mℓ of each of whole blood samples having different Hct values was spotted on the blood cell-filtering layer of the analytical element. After one minute, the each blood cell-filtering layer was peeled off and incubated at 37^{o}C for 6 minutes. Reflection density (OD, measurement wavelength: 540 nm) was measured on the side of the transparent support. The results are shown in Table 2.

**Table 2**

| (Results of assay of bilirubin) | | | | |
|---|---|---|---|---|
| BIL concentration | Hct (%) | OD (%) | Assayed value (mg/dℓ) | Reference value (mg/dℓ) |
| level I | 35 | 0.525 | 17.2 | 17.1 |
| | 55 | 0.524 | 17.1 | 17.0 |
| | 65 | 0.522 | 17.0 | 17.1 |
| level II | 40 | 0.388 | 9.9 | 9.7 |
| | 53 | 0.385 | 9.7 | 9.8 |
| | 60 | 0.387 | 9.8 | 9.8 |
| level III | 30 | 0.323 | 6.4 | 6.3 |
| | 54 | 0.320 | 6.2 | 6.3 |
| | 62 | 0.321 | 6.3 | 6.3 |

In Table 2, BIL concentration represents bilirubin concentration, the assayed value represents BIL concentration obtained from the conversion of reflection density (OD) measured by using the above analytical element for the analysis of whole blood, and the reference value is an assayed value obtained by a wet analytical method (J-G method) using the known plasma as a sample.

It has been found from the above results that when the analysis is done using the analytical element of the present invention, the whole blood samples having a bilirubin content on the same level give approximately the same absorbance and assayed values, irrespective of variation of the Hct value. Further, it has been found that the assayed values well agree with the values obtained by assaying bilirubin through the wet method using plasma obtained by filtering the whole blood sample, and hence, the analytical element of the present invention is very useful for the analysis of whole blood.

### Example 3

The liquid sample-metering function of the spreading layer of the analytical element for the analysis of whole blood prepared in Example 2, was tested. It was observed that low-concentration samples in an amount between 8 to 12 µℓ gave the same assayed value of 1.0 mg/dℓ, and medium-concentration samples in an amount of 8 uℓ gave the assayed value of 5.6 mg/dℓ, in an amount of 9 uℓ gave the value of 5.7 mg/dℓ, in an amount of 11µℓ gave the value of 5.6 mg/dℓ and in an amount of 12 uℓ gave the value of 5.6 mg/dℓ. Accordingly, it was confirmed that the liquid sample-metering function of the analytical element of the present invention was satisfactory.

### Example 4

According to the Example 6 of Japanese Patent Provisional Publication No. 59(1984)-12355, an indicator-containing layer (dry film thickness: 8 µm) was formed on a transparent polyethylene terephthalate support (thickness: 180 µm), a liquid blocking layer was then laminated thereon under pressure, and further a substrate-decomposing material-containing layer (dry film thickness: 10 µm), a light-reflecting layer (dry film thickness: 6 µm) and then a bonding layer (dry film thickness: 2 µm) were formed thereon in turn. Finally, a spreading layer composed of a broad weave cotton cloth was laminated under pressure to prepare an integral multilayer analytical element for the analysis of BUN (urea nitrogen). Each of the coating solutions for the indicator-containing layer, the substrate-decomposing material-containing layer, the light-reflecting layer and the bonding layer had the following composition. The methods for the preparation of the liquid-blocking layer were also described below.

| Coating solution for indicator-containing layer | |
|---|---|
| Bromocresol Green | 8.1 g. |
| Vinyl acetate-acrylate ester copolymer latex (Cebian A-5821 manufactured by Daisel Co., Ltd.) | 510 g. |
| Citric acid | 4.5 g. |
| Water | 550 mℓ. |

### Liquid-blocking layer

A membrane filter FM-300 (thickness: 140 µm, voids: about 85%, minimum pore diameter: 3 µm, manufactured by Fuji Photo Film Co., Ltd.) produced from cellulose di- and triacetate was immersed in a hexane solution of 2% silicone (Toray silicone SH-8011, manufactured by Toray Co., Ltd.) and then dried to prepare the desired layer.

| Coating solution for substrate-decomposing material-containing layer | |
|---|---|
| Deionized gelatin | 9.0 g/m² |
| p-Nonylphenoxy polyglycidol | 0.2 g/m² |
| Tetrasodium ethylenediamine-tetraacetate | 3.45 g/m² |
| Sodium N-2-hydroxyethylpiperazine-N'-3-propane sulfonate (EPPS) | 3.45 g/m² |
| Disodium hydrogenphosphate | 0.115 g/m² |
| 1.4-Dithiothreitol [3483-12-3] | 0.9 mg/m² |
| Urease | 36,000 U/m² |
| Bisvinylsulfonyl methyl ether | 54 mg/m² |
| The above solution was buffered to pH 8.0. | |

| Coating solution for light-reflecting layer | |
|---|---|
| Fine titanium dioxide | 40 g. |
| Gelatin | 40 g. |
| p-Nonylphenoxy polyglycidol | 1.5 g. |
| Water | 400 mg. |

| Coating solution for bonding layer | |
|---|---|
| Gelatin | 25 g. |
| Water | 500 mg. |
| p-Nonylphenoxy polyglycidol | 1.5 g. |

A glass fiber filter GB-100R (a disc of 7 mm in diameter, manufactured by Toyo Filter Paper Co., Ltd.) was placed on the analytical element by slightly bonding two portions on the periphery of the glass fiber disc to the element with an α-cyanoacrylate adhesive, to form a blood cell-filtering layer. Thus, an analytical element for the analysis of BUN of whole blood according to the present invention was obtained.

25 µℓ of heparin-treated whole blood as a sample was spotted on the blood cell-filtering layer. Within one minute after spotting, 9.5 µℓ of plasma was diffused into the spreading layer. The hemolyzation and the flow-out of erythrocyte were not observed at all.

Further, the analysis of the spread sample was made according to the measurement method of Example 6 of the aforementioned Japanese Patent Provisional Publication No. 59(1984)-12355. Separately, 9.5 µℓ of plasma was separated from the same whole blood sample by centrifugation and spotted on the multilayer analytical element for the analysis of BUN, which did not have any glass fiber filter. Both experiments gave approximately the same results.

### Example 5 (Reference Example)

Various glass fiber filters were tested to evaluate the separation of plasma from blood cell in the whole blood sample.

Discs were punched from various glass fiber filters (set forth in Table 3) manufactured by Toyo Filter Paper Co., Ltd. and placed on a sheet having the liquid sample-metering function for cellulose thin-layer chromatography (DC-Plastikfolten Cellulose Art. 5577, manufactured by Merck Inc.).

A heparin-treated whole blood sample was spotted on each of them. After one minute, the glass fiber filter was removed, and a circle formed by the spreading of only plasma over the chromatography sheet was measured by means of a planimeter.

**Table 3**

| Filter No. | Thickness mm | Density g/cm³ | Diameter mm | Volume mm³(V) | Absorption amount mm³(Vb) |
|---|---|---|---|---|---|
| 1 GA100 | 0.45 | 0.24 | 7 | 17.3 | 15.4 |
| 2 GA100 | 0.45 | 0.24 | 4.5 | 7.2 | 6.4 |
| 3 GA200 | 0.75 | 0.23 | 6 | 21.0 | 18.8 |
| 4 GB100R | 0.40 | 0.24 | 7 | 15.4 | 13.6 |

On the other hand, each of 3, 5, 7, 9, 11, 13, and 15 µℓ of serum was separated by centrifugation and spotted on the same chromatographic sheet. The resulting spread area was measured likewise and found to be 0.50, 0.88, 1.19, 1.57, 1.83, 2.23, and 2.57 cm², respectively. Thus, it was confirmed that said chromatographic sheet had the liquid sample-spreading function, and a calibration curve (plasma amount = 5.83 x spread area) was obtained. The amount of spread plasma was calculated from the spread area using said calibration curve. The results are given in Table 4.

**Table 4**

| Filter No. | Spotted amount µℓ | Amount of Plasma mm³(Vs) | Absorption amount mm³(Vb) | Vs/Vb % |
|---|---|---|---|---|
| 1 | 40 | 13.2 | 15.4 | 85 |
| 2 | 20 | 8.2 | 6.4 | 128 |
| | 30 | 8.6 | 6.4 | 134 |
| | 40 | 12.3 | 6.4 | 192 |
| 3 | 30 | 6.9 | 18.8 | 37 |
| | 40 | 12.5 | 18.8 | 66 |
| | 50 | 15.0 | 18.8 | 80 |
| 4 | 30 | 10.9 | 13.6 | 80 |
| | 40 | 14.2 | 13.6 | 104 |

### Example 6 (Reference Example)

The relationship between the hematocrit value of the whole blood sample and the amount of plasma filtered through the filter was examined.

A glass fiber filter GB-100R(diameter: 7 mm, absorption amount [Vb] = 13.6, manufactured by Toyo Filter Paper Co., Ltd.) was used, and 25 µℓ of the whole blood sample was spotted. A similar experiment to that of Example 5 was carried out. The results are shown in Table 5.

**Table 5**

| Hct value | Amount of Plasma (found) (Vs)µℓ | Amount of Plasma (calculated) (Vc)µℓ | Vs/Vc (%) | Vs/Vb (%) |
|---|---|---|---|---|
| 30 | 11 | 17.5 | 63 | 81 |
| 50 | 9.5 | 12.5 | 76 | 70 |
| 70 | 7.6 | 7.5 | 101 | 56 |

## Claims

1. An analytical element for the analysis of a whole blood sample; comprising
a spreading layer (21) capable of metering a liquid sample, a detection layer (22) containing an analytical reagent and a water-impermeable, light-transmissive support (23), arranged in this order, or
comprising a spreading layer (21) capable of metering a liquid sample, which contains an analytical reagent, and a water-impermeable, light-transmissive support (23), characterized in that said spreading layer (21) is composed of woven cloth or knitted cloth and said spreading layer (21) has a blood cell-filtering layer (24) directly on its surface, said blood cell-filtering layer being fixed onto the surface of the spreading layer by pressing or bonding peripheral edge area of the blood cell-filtering layer to the spreading layer in such a manner that the flow of the liquid sample is not interfered by the pressed or bonded area.

2. The analytical element as claimed in claim 1, wherein said spreading layer (21) is composed of broad cloth.

3. The analytical element as claimed in claim 1, wherein said blood cell-filtering layer (24) contains a light-blocking material.

4. The analytical element as claimed in claim 1, wherein said blood cell-filtering layer is a sheet-form filter composed of a glass fiber.

5. The analytical element as claimed in claim 1, wherein said blood cell-filtering layer (24) has a thickness in the range of 100 to 2,000 µm.

## Patentansprüche

1. Analytisches Element zur Analyse einer Vollblutprobe, umfassend
eine Ausbreitungsschicht (21) die imstande ist, eine flüssige Probe zu messen, eine Nachweisschicht (22), die ein analytisches Reagens enthält, sowie einen wasserundurchlässigen, lichtdurchlässigen Träger (23), angeordnet in dieser Reihenfolge, oder umfassend eine Ausbreitungsschicht (21) die imstande ist, eine flüssige Probe zu messen, wobei die Schicht ein analytisches Reagens enthält, und einen wasserundurchlässigen, lichtdurchlässigen Träger (23), dadurch gekennzeichnet, daß die Ausbreitungsschicht (21) zusammengesetzt ist aus gewebtem Tuch oder gewirktem Tuch und die Ausbreitungsschicht (21) direkt auf ihrer Oberfläche eine blutzellenfilternde Schicht (24) aufweist, wobei die blutzellenfilternde Schicht auf der Oberfläche der Ausbreitungsschicht befestigt ist durch Andrücken oder Binden der peripheren Kantenfläche der blutzellenfilternden Schicht an die Ausbreitungsschicht derart, daß der Fluß der flüssigen Probe durch die angedrückte oder gebundene Fläche nicht gestört wird.

2. Analytisches Element nach Anspruch 1, wobei die Ausbreitungsschicht (21) aus feinem Wollstoff zusammengesetzt ist.

3. Analytisches Element nach Anspruch 1, wobei die blutzellenfilternde Schicht (24) ein lichtsperrendes Material enthält.

4. Analytisches Element nach Anspruch 1, wobei die blutzellenfilternde Schicht ein Schichtenformfilter ist, zusammengesetzt aus Glasfaser.

5. Analytisches Element nach Anspruch 1, wobei die blutzellenfilternde Schicht (24) eine Dicke im Bereich von 100 bis 2000 µm aufweist.

## Revendications

1. Un élément analytique pour l'analyse d'un échantillon de sang entier; comprenant
une couche d'étalement (21) capable de doser un échantillon de liquide, une couche de détection (22) contenant un réactif analytique et un support (23) transmettant la lumière imperméable à l'eau, agencées dans cet ordre, ou comprenant une couche d'étalement (21) capable de doser un échantillon de liquide, qui contient un réactif analytique, et un support (23) transmettant la lumière et imperméable à l'eau, caractérisé en ce que ladite couche d'étalement (21) est composée d'étoffe tissée ou d'étoffe tricotée et ladite couche d'étalement (21) a une couche filtrante des cellules sanguines (24) directement sur sa surface, cette couche filtrante des cellules sanguines étant fixée sur la surface de la couche d'étalement par pressage ou liaison de la surface du bord périphérique de la couche filtrante des cellules sanguines à la couche d'étalement d'une manière telle que l'écoulement de l'échantillon de liquide n'est pas interféré par la surface pressée ou liée.

2. L'élément analytique selon la revendication 1, selon laquelle ladite couche d'étalement (21) est composée d'étoffe large.

3. L'élément analytique selon la revendication 1, selon laquelle ladite couche filtrante des cellules sanguines (24) contient un matériau de blocage de la lumière.

4. L'élément analytique selon la revendication 1, selon laquelle ladite couche filtrante des cellules sanguines est un filtre en forme de feuille composé de fibres de verre.

5. L'élément analytique selon la revendication 1, selon laquelle ladite couche filtrante des cellules sanguines (24) a une épaisseur dans l'intervalle de 100 à 2 000 µm.
